Europäisches Patentamt

European Patent Office

Office européen des brevets

(19)

(11) Veröffentlichungsnummer: **0 044 436**

**A1**

(12) # EUROPÄISCHE PATENTANMELDUNG

(21) Anmeldenummer: 81104947.7

(22) Anmeldetag: 26.06.81

(51) Int. Cl.³: **G 01 R 13/22**
G 01 R 13/34, G 01 R 29/02
A 61 B 5/04

(30) Priorität: 02.07.80 US 165169
02.07.80 US 165172

(43) Veröffentlichungstag der Anmeldung:
27.01.82 Patentblatt 82/4

(84) Benannte Vertragsstaaten:
DE FR GB

(71) Anmelder: Hewlett-Packard Company
1501 Page Mill Road
Palo Alto California 94304(US)

(72) Erfinder: Beebe, Richard P.
82 Wyman Road
Billerica Massachusetts 01821(US)

(74) Vertreter: Schulte, Knud, Dipl.-Ing.
c/o Hewlett-Packard GmbH Europ. Patent- und
Lizenzabteilung Postfach 1430 Herrenberger Strasse 130
D-7030 Böblingen(DE)

(54) Schaltungsanordnung zum Erzeugen von Anzeigesignalen.

(57) Aus einer Folge von Eingangssignalen, welche Herz-schrittmachersignale oder andere relativ schmale Spitzen-wertsignale vergleichsweise großer Amplitude aufweist, wird zur Anzeige eine geringere Anzahl von Signalen durch eine Schaltungsanordnung derart ausgewählt, daß die Spitzenwertsignale in der Anzeige erkennbar bleiben. Hierzu werden die Eingangssignale in gleiche, einander ausschlie-ßende Gruppen von Signalen unterteilt, und als Anzeigesign-al wird dasjenige Signal einer Gruppe ausgewählt, dessen Amplitude sich betragsmäßig am meisten von einem für die vorherige Gruppe ausgewählten Referenzwert unterschei-det. Dieser Referenzwert kann der Durchschnittswert der vorherigen Gruppe oder der auf gleiche Weise wie der Anzeigewert selbst ermittelte Wert der vorherigen Gruppe sein.

./...

EP 0 044 436 A1

_Fig. 4._

Int. Az.: Case 1442/43                    15. Juni 1981

SCHALTUNGSANORDNUNG ZUM ERZEUGEN VON ANZEIGESIGNALEN

Die Erfindung betrifft eine Schaltungsanordnung zum Erzeugen von Anzeigesignalen gemäß dem Oberbegriff von Patentanspruch 1.

Insbesondere Vorrichtungen zum Überwachen physiologischer Funktionen,
beispielsweise zur Überprüfung der elektrischen Aktivität des Herzens,
erzeugen häufig digitale Abtastsignale mit einer Frequenz, die für die
Anzeige durch Kathodenstrahlröhren und andere Anzeigevorrichtungen unnötig groß ist. Beispielsweise kann es vorkommen, daß die Abtastsignale
alle zwei Millisekunden auftreten und daß die Anzeigevorrichtung nur in
der Lage ist, alle acht Millisekunden ein Abtastsignal darzustellen, so
daß eine Datenreduktion im Verhältnis 4:1 erforderlich ist. Eine Möglichkeit, die Datensignale zu reduzieren, bestünde darin, den Mittelwert
jeder Gruppe von vier Abtastsignalen zu bestimmen. Dadurch würde jedoch
die Amplitude von schmalen Herzschrittmacherimpulsen wesentlich geschwächt, obgleich diese sehr wichtig sind und nicht in Rauschsignalen
untergehen dürfen.

Der Erfindung liegt somit die Aufgabe zugrunde, eine Schaltungsanordnung
gemäß dem Oberbegriff von Anspruch 1 derart zu verbessern, daß aus
digitalen Eingangssignalen, welche unter anderem relativ schmale Signale
mit relativ hoher Amplitude aufweisen, eine verminderte Anzahl von
Ausgangssignalen abgeleitet wird, welche zur Anzeige ausgewertet werden.

Ausgehend von einer Schaltungsanordnung gemäß dem Oberbegriff wird diese
Aufgabe gelöst durch die Merkmale des Kennzeichens von Patentanspruch 1.

−1−

In einer Ausgestaltung des Grundgedankens der Erfindung wird die absolute Differenz zwischen dem Wert jedes (Abtast-)Signales in einer Gruppe von (Abtast-)Signalen und dem Durchschnittswert der vorherigen Gruppe von (Abtast-)Signalen bestimmt und dasjenige (Abtast-)Signal als Anzeigesignal ausgewählt, welches die größte absolute Differenz aufweist.

Gemäß einer anderen Ausführungsform der Erfindung wird dasjenige Abtastsignal jeder Gruppe als Anzeigesignal ausgewählt, dessen Amplitude sich am meisten von dem Abtastsignal unterscheidet, welches in der vorhergehenden Gruppe auf die gleiche Weise als Anzeigesignal ausgewählt wurde.

Es versteht sich, daß bei beiden Ausführungsformen der Vergleich der einzelnen Signale einer Gruppe mit einem aus der vorherigen Gruppe von Datensignalen abgeleiteten Referenzwert sowohl für alle Signale einer Gruppe gleichzeitig als auch nacheinander durchgeführt werden kann, obgleich nachfolgend nur Ausführungsformen erläutert werden, bei denen dieser Vergleich parallel für alle Signale einer Gruppe durchgeführt wird.

Nachfolgend werden zwei bevorzugte Ausführungsbeispiele der Erfindung anhand der Zeichnungen erläutert; es zeigen:

Figur 1 eine Anzahl von (Abtast-)Signalen, aus denen ein Elektrokardiogramm abgeleitet wird, wobei in jeder aufeinanderfolgenden Gruppe vier Signale dargestellt sind;

Figur 2 die Anzeigesignale, welche von den Eingangssignalen gemäß Figur 1 abgeleitet würden, wenn jedes Anzeigesignal der Durchschnittswert jeder exklusiven, d.h. also Signale einer anderen Gruppe ausschließenden Gruppe von vier Datensignalen wäre;

Figur 3 die Abtastsignale aus Figur 1, welche gemäß einer Ausführungsform der Erfindung als Anzeigesignale ausgewählt werden;

Figur 4 ein Blockdiagramm einer Schaltungsanordnung, welche aus den Eingangssignalen die Anzeigesignale derart auswählt, daß die absolute Differenz zwischen allen Datensignalen einer Gruppe und dem Durchschnittswert der Datensignale der vorhergehenden Gruppe gleichzeitig gebildet wird, bevor das Datensignal mit der größten absoluten Differenz als Anzeigesignal ausgewählt wird;

Figur 4A Signale, welche beim Betrieb der Schaltungsanordnung gemäß Figur 4 auftreten;

Figur 5 ein Flußdiagramm, aus dem eine Weise hervorgeht, wie die aus den Eingangssignalen abgeleiteten Anzeigesignale durch einen Prozessor oder Computer erfindungsgemäß ausgewählt werden können;

Figur 6 ein aus Eingangssignalen gebildetes Elektrokardiogramm;

Figur 7 ein Elektrokardiogramm, welches unter Verwendung von Durchschnittssignalen jeder Gruppe von vier Eingangssignalen abgeleitet wurde;

Figur 8 ein Elektrokardiogramm, welches gebildet wurden, indem gemäß einer Ausführungsform der Erfindung aus jeder Gruppe von vier Eingangs-Abtastsignalen eines als Anzeigesignal ausgewählt wurde;

Figur 9 ein Blockdiagramm einer Schaltungsanordnung, welche aus den Eingangssignalen die Anzeigesignale deart auswählt, daß die Absolutwerte der Differenzen zwischen den Datenwerten einer Gruppe und dem Datenwert der vorherigen Gruppe gleichzeitig abgeleitet werden, bevor das Abtastsignal mit der größten absoluten Differenz als Anzeigewert ausgewählt wird;

Figur 9A schematisch die Folgen von Eingangs- und Ausgangs-Abtastimpulsen, die in der Schaltungsanordnung gemäß Figur 9 auftreten, sowie die abgetasteten Eingangssignale.

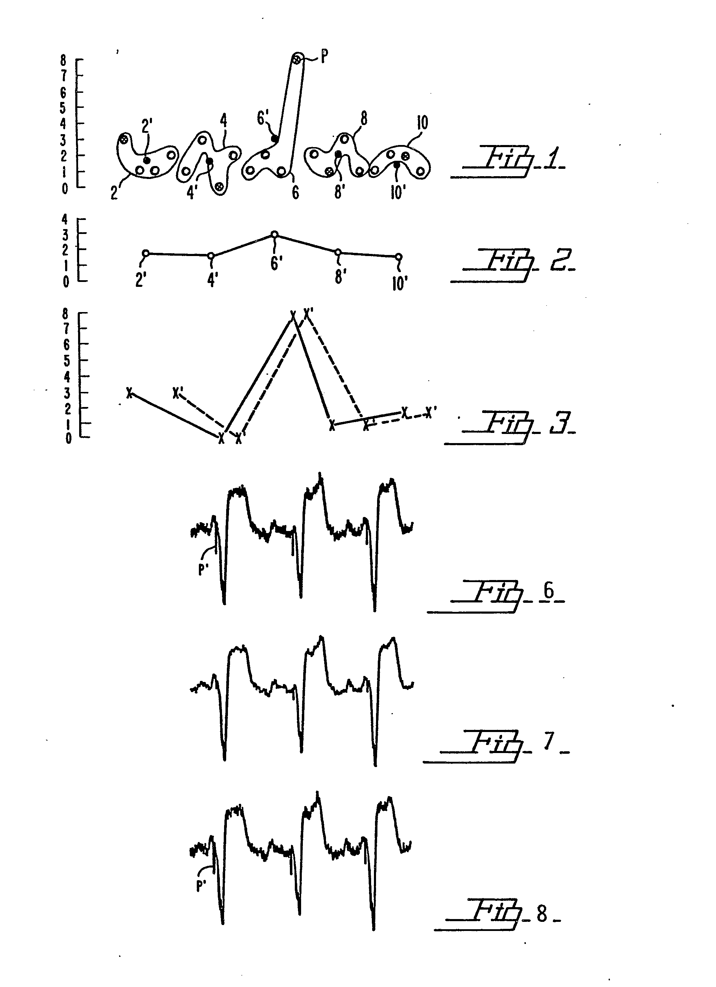

In Figur 1 ist jedes Abtastsignal bezeichnet mit "0"; nachfolgende, sich gegenseitig ausschließende Gruppen von vier Datensignalen sind durch getrennte Linien 2, 4, 6, 8 und 10 zusammengefaßt und der Durchschnittswert der Datensignale jeder Gruppe ist durch entsprechende Punkte 2', 4', 6', 8' und 10' bezeichnet. Der Datenpunkt P in der Gruppe 6 stellt den Herzschrittmacherimpuls dar.

Figur 2 stellt das Elektrokardiogramm dar, welches sich ergäbe, falls die Eingangssignale reduziert werden, indem der Durchschnittswert jeder Gruppe von vier Abtastsignalen, d.h. die Punkte 2', 4', 6', 8' und 10' als Anzeigesignale ausgewertet würden. Dabei ist zu beachten, daß der Schrittmacherimpuls P dargestellt würde durch das Anzeigesignal 6' der Gruppe 6 und somit wesentlich abgeschwächt würde.

Erfindungsgemäß wird ein Abtastsignal in jeder der Gruppen 2, 4, 6, 8 und 10 als Anzeigesignal ausgewertet. Gemäß einer Ausführungsform ist dieses das Eingangs-Abtastsignal, das sich von dem Durchschnittswert der vorhergehenden Gruppe von Abtastsignalen um den größten absoluten Betrag unterscheidet. Die Abtastsignale, welche als Anzeigesignale erfindungsgemäß ausgewählt werden, sind durch ein "X" bezeichnet. Sie sind in Figur 3 mittels einer durchgehenden Linie verbunden. Daraus ergibt sich, daß der Schrittmacherimpuls P als Anzeigesignal erhalten wurde. Da das Anzeigesignal aus den Signalen einer Gruppe erst bestimmt werden kann, nachdem alle Signale dieser Gruppe untersucht wurden, wird das Abtastsignal, welches als Anzeigesignal ausgewählt wurde, der Anzeigeeinrichtung erst zu einem Zeitpunkt zwischen dem letzten Abtastsignal einer Gruppe und dem ersten Abtastsignal der nächsten Gruppe zugeführt, wobei sich diese Zeitverschiebung in Figur 3 durch die mit X' bezeichneten und durch unterbrochene Linien verbundenen Punkte ergibt.

Die Schaltungsanordnung gemäß Figur 4 wählt das Datensignal aus jeder Gruppe von vier Datensignalen, welches erfindungsgemäß als Abtastsignal verwendet wird. Hierzu wird in folgender Weise der Durchschnittswert jeder Gruppe von Datensignalen gebildet. Analoge Signale, beispielsweise die Ausgangssignale eines EKG-Gerätes, werden von einer Signalquelle 12 einem A/D-Umsetzer 14 zugeführt, der digitalisierte Abtastsignale mit einer Frequenz und Dauer ergibt, die durch Triggerimpulse S gemäß Figur 4A bestimmt werden. Obgleich nur eine Datenleitung dargestellt ist, könnte natürlich jede gewünschte Anzahl von Leitungen vorgesehen werden. Die Triggerimpulse S werden durch einen Impulsgenerator 16 abgegeben, der ein digitaler Zähler sein kann. Die digitalisierten Abtastsignale am Ausgang des A/D-Umsetzers 14 werden in einem Speicher $L_1$ eingespeichert, der in Reihe mit den Speichern $L_2$, $L_3$ und $L_4$ geschaltet ist. Der Ausgang Q jedes dieser Speicher ist mit dem Eingang D des nächsten Speichers in Reihe geschaltet und bildet somit ein Schieberegister. Alle Speicher werden durch Impulse S aktiviert. Die Eingänge einer Addierschaltung 18 sind jeweils mit den Aus-

gängen Q der Speicher $L_1$ und $L_2$ verbunden. Die Eingänge einer Addierschaltung 20 sind entsprechend mit dem Ausgang einer Addierschaltung 18 und dem Ausgang Q des Speichers $L_3$ verbunden. Die Eingänge einer Addierschaltung 22 sind mit dem Ausgang einer Addierschaltung 20 und dem Q-Ausgang des Speichers $L_4$ verbunden. Der Ausgang einer Addierschaltung 22 ist mit Ausnahme der beiden bits mit der tiefsten Stellenwertigkeit verbunden mit dem Eingang D eines Speichers $L_5$, und das Ausgangssignal der Addierschaltung 22 wird durch vier geteilt. Somit treten nach vier Taktimpulsen S einer ersten Gruppe I gemäß Figur 4A die nachfolgenden Abtastsignale A, B, C und D dieser Gruppe an den Ausgängen der Speicher $L_4$, $L_3$, $L_2$ und $L_1$ auf, und für deren Summen repräsentative Signale treten am Ausgang der Addierschaltung 22 auf. Ein Viertel dieser Summe bzw. der Durchschnittswert der Abtastsignale A, B, C und D wird dem Eingang D des Speichers $L_5$ zugeführt. Dieser Durchschnittswert wird zum Ausgang Q des Speichers $L_5$ durch einen Impuls O gemäß Figur 4A übertragen, der durch den Impulsgenerator 16 erzeugt wird. Da die Pulse O zwischen dem letzten Impuls S einer Gruppe und dem ersten Impuls S der nächsten Gruppe auftraten, ist der Durchschnittswert am Ausgang Q des Speichers $L_5$ während der Gruppe II der Durchschnittswert der Abtastsignale der Gruppe I.

Die Übertragung des in noch zu beschreibender Weise ausgewählten Abtastwertes an eine Ausgangssammelleitung 24 und eine Anzeigeeinrichtung 25 erfolgt so: die Abtastwerte am Ausgang Q der Speicher $L_1$, $L_2$, $L_3$ und $L_4$ treten auch entsprechend an den Eingängen D der Speicher $L_6$, $L_7$, $L_8$ und $L_9$ aufgrund der zwischen den Speichern bestehenden Verbindungen auf, und alle Q-Ausgänge der Speicher $L_6$, $L_7$, $L_8$ und $L_9$ sind mit der Ausgangssammelleitung 24 für Anzeigesignale verbunden. Die Ausgänge von UND-Gliedern 6', 7', 8' und 9' sind jeweils mit den Triggereingängen der Speicher $L_6$, $L_7$, $L_8$ und $L_9$ verbunden, und ein Eingang jedes UND-Gliedes ist durch eine Sammelleitung 26 mit dem Ausgang des Impulsgenerators 16 verbunden, an dem die Impulse O gemäß Figur 4A auftreten. Einer der anderen Eingänge d', c', b' und a' der entsprechenden UND-Glieder 6', 7', 8' und 9' hat den Signalpegel H während eines Impulses O, so daß der Ausgang dieses UND-Gliedes den Signalpegel H hat und den Speicher triggert, mit dem dieses UND-Glied verbunden ist. Das Abtastsignal am Eingang dieses Speichers ist das Anzeigesignal und es wird an den Q-Ausgang und über die Ausgangssammelleitung 24 an die Anzeigeeinrichtung 25 übertragen.

Die Ableitung der absoluten Werte der Differenzen zwischen jedem Abtastsignal einer Gruppe und dem Durchschnittswert der Abtastsignale der vorhergehenden Gruppe erfolgt durch die folgenden Schaltungsgruppen. Ein Satz von Eingängen von Subtrahiergliedern 26, 28, 30 und 32 ist mit den Q-Ausgängen der Speicher $L_1$, $L_2$, $L_3$ und $L_4$ verbunden, an denen die Abtastwerte auftreten, und die anderen Eingänge dieser Subtrahierglieder sind mit dem Q-Ausgang des Speichers $L_5$ verbunden, an dem der Durchschnittswert der vorhergehenden Gruppe von Abtastsignalen auftritt. Wenn somit die Abtastsignale D', C', B' und A' der Gruppe II entsprechend an den Q-Ausgängen der Speicher $L_1$, $L_2$, $L_3$ und $L_4$ auftreten, treten die Differenzwerte zwischen diesen Abtastsignalen und dem Durchschnittswert der Abtastsignale der Gruppe I entsprechend an den Ausgängen der Subtrahierglieder 26, 28, 30 und 32 auf. Die absoluten Werte dieser Differenzen, $|D'-\overline{X}|$, $|C'-\overline{X}|$, $|B'-\overline{X}|$ und $|A'-\overline{X}|$ werden durch Schaltungen 26', 28', 30' und 32' zur Bildung des Absolutwertes abgeleitet, die mit den Ausgängen der Subtrahierglieder 26, 28, 30 bzw. 32 verbunden sind.

Die Auswahl des Abtastwertes einer Gruppe mit dem größten absoluten Differenzbetrag zum Durchschnittswert der vorhergehenden Gruppe erfolgt durch folgende Schaltung: der Ausgang des Schaltkreises 26 ist mit dem X-Eingang eines Komparators 34 sowie mit einer Eingangsklemme l eines Schalters $S_1$ verbunden, und der Ausgang des Schaltkreises 28' ist mit dem Eingang Y des Komparators 34 sowie mit einer Eingangsklemme r des Schalter $S_1$ verbunden. Der Ausgang 36 des Komparators 34 ist mit einer Steuerklemme 38 des Schalters $S_1$ verbunden. Wenn der Wert am Eingang X des Komparators 34 kleiner als der Wert an seinem Y-Eingang ist, hat das Signal am Ausgang 36 den Signalpegel H, und der Ausgang 0 des Schalters $S_1$ ist mit der Eingangsklemme r verbunden. Der Ausgang des Schaltkreises 30' ist mit dem Eingang X eines Komparators 40 sowie mit einer Eingangsklemme l eines Schalters $S_2$ verbunden, und der Ausgang des Schaltkreises 32' ist mit dem Y-Eingang des Komparators 40 sowie mit einer Eingangsklemme r des Schalters $S_2$ verbunden. Der Ausgang 42 des Komparators 40 ist mit einer Steuerklemme 44 des Schalters $S_2$ verbunden. Wenn der Wert des Eingangs X des Komparators 40 kleiner als der Wert des Signales an dessen Y-Eingang ist, hat das Signal am Ausgang 42 den Signalpegel H und der Ausgang 0 des Schalters $S_2$ ist mit der Eingangsklemme r verbunden. Die Ausgänge 0 der Schalter $S_1$ und $S_2$ sind jeweils mit den Eingängen X und Y eines Komparators 46 verbunden, der einen Ausgang 48 hat. Im Fall der anderen Komparatoren, hat der Ausgang 48 den Signalpegel H, falls der Wert am Eingang X kleiner als der Wert am Eingang Y ist.

Die jetzt zu beschreibenden Logikschaltkreise verarbeiten die Ausgangssignale der Komparatoren 34, 40 und 46 und heben den Signalpegel eines der entsprechenden Eingänge a', b', c' und d' der UND-Glieder 9', 8', 7' und 6' so, daß dadurch der zugeordnete Speicher getriggert wird und das Abtastsignal an seinem Eingang an die Ausgangssammelleitung 24 überträgt, wenn der Ausgangsabtastimpuls 0 auftritt. Dieses Datensignal ist dann das Anzeigesignal. Die Ausgänge der UND-Glieder 9", 8", 7" und 6" sind jeweils mit den Eingängen A', B', C' und D' der UND-Glieder 9', 8', 7' und 6' verbunden. Die Eingänge des UND-Gliedes 9" sind jeweils mit den Ausgängen 42 und 48 der Komparatoren 40 und 46 verbunden. Die Eingänge des UND-Gliedes 8" sind jeweils mit dem Ausgang 48 des Komparators 46 und dem Ausgang 42 des Komparators 40 über einen Umkehrverstärker 50 verbunden. Die Eingänge des UND-Gliedes 7" sind jeweils mit dem Ausgang 36 des Komparators 34 und über einen Umkehrverstärker 52 mit dem Ausgang 48 des Komparators 46 verbunden. Die Eingänge des UND-Gliedes 6" sind jeweils mit dem Ausgang 36 des Komparators 34 über einen Umkehrverstärker 54 und über den Umkehrverstärker 52 mit dem Ausgang 48 des Komparators 46 verbunden. Der Betrieb der Schaltung gemäß Figur 4 zur Auswahl eines Abtastsignales der Gruppe II wird nun in Verbindung mit Figur 4A erläutert. Es wird davon ausgegangen, daß die Werte der Abtastsignale während aufeinanderfolgender, sich gegenseitig ausschließender Signalgruppen I bzw. II in der Reihenfolge A, B, C und D bzw. A', B', C' und D' gemäß Figur 4A auftreten. Nachdem das vierte Abtastsignal D der Gruppe I erhalten wurde, treten die Abtastsignale A, B, C und D an den Ausgängen der entsprechenden Speicher $L_4$, $L_3$, $L_2$

und $L_1$ auf, und am Ausgang der Addierschaltung 22 erscheint ein Signal mit der Summe A, B, C und D. Da die beiden bits mit der geringsten Stellenwertigkeit dieser Summe nicht zum D-Eingang des Speichers $L_5$ übertragen werden, ist der Wert am D-Eingang der Durchschnitt der Datensignale der Gruppe I oder (A+B+C+D)/4 entsprechend der unterbrochenen Linie 56. Dies ist der Zustand, wenn ein Taktimpuls 58 der Gruppe I auftritt. Der Ausgangsimpuls 58 triggert den Speicher $L_5$ und bewirkt, daß der Durchschnittswert an dessen D-Eingang an dessen Q-Ausgang und damit an einen Eingang jeder der Subtrahierschaltungen 26, 28, 30 und 32 übertragen wird. Dieser Durchschnittswert bleibt am Q-Ausgang des Speichers $L_5$ während der Aufnahme der nächsten Gruppe II von Abtastsignalen A', B', C' und D' und bis der Ausgangstaktimpuls 60 der Gruppe II erscheint.

Nachdem die Abtastsignale A', B', C' und D' der Gruppe II angekommen sind und kurz bevor der Taktimpuls 60 auftritt, sind die Werte an den Ausgängen der Speicher $L_4$, $L_3$, $L_2$ und $L_1$ A', B', C' bzw. D' entsprechend der Zeichnung. Diese Werte werden Eingängen der Subtrahierschaltungen 32, 30, 28 und 26 zugeführt, und der Durchschnittswert $\overline{X}$ = (A+B+C+D)/4 der Abtastsignale der Gruppe I wird anderen Eingängen vom Ausgang Q des Speichers $L_5$ zugeführt, wie schematisch durch die unterbrochene Linie 56 in Figur 4A dargestellt ist, so daß die Ausgänge der Subtrahierschaltungen Signale entsprechend den Werten A'-$\overline{X}$, B'-$\overline{X}$, C'-$\overline{X}$ und D'-$\overline{X}$ führen. Die absoluten Werte $|A'-\overline{X}|$ und $|B'-\overline{X}|$ werden durch die Schaltkreise 32' und 30' abgeleitet, die den Eingängen Y und X des Komparators 40 zugeführt werden. Da entsprechend der Darstellung in Figur 4A gilt $|A'-\overline{X}| > |B'-\overline{X}|$ hat der Ausgang des Komparators 40 den Signalpegel H und der Ausgang des Schalters $S_2$ ist mit der Eingangsklemme r verbunden, um den Wert $|A'-\overline{X}|$ an den Y-Eingang des Komparators 46 zu übertragen.

Die Ausgänge der Subtrahierglieder 26 bzw. 28 haben die Signale D'-$\overline{X}$ und C'-$\overline{X}$, und deren absolute Werte $|D'-\overline{X}|$, und $|C'-\overline{X}|$ werden durch die Subtrahierglieder 26' bzw. 28' abgeleitet und den X- und Y-Eingängen des Komparators 34 zugeführt. Wegen der Relation

$|D'-\overline{X}| > |C'-X|$ hat der Ausgang des Komparators 34 den Signalpegel L, damit der Ausgang 0 des Schalters $S_1$ mit der Eingangsklemme 1 verbunden ist und dadurch den Wert $|D'-\overline{X}|$ an den X-Eingang des Komparators 46 überträgt. In diesem Beispiel ist der Wert $|A'-\overline{X}|$ größer als der Wert $|B'-\overline{X}|$, so daß der Ausgang des Komparators 46 den Signalpegel L in der angegebenen Weise führt.

Bezeichnet man symbolisch die Signalzustände an den Ausgängen 36, 48 und 42 der Komparatoren 34, 46 und 40 entsprechend mit (-), (-) und (+), so ergeben sich die in Figur 4A dargestellten Signalpegel an den Eingängen der UND-Glieder 6", 7", 8" und 9", so daß der Ausgang des UND-Gliedes 6", der mit dem Eingang d' des UND-Gliedes 6' verbunden ist, der einzige Ausgang ist, der den Signalpegel H führt. Dadurch kann der Speicher $L_6$ den Wert des Abtastsignales D' vom Eingang D an den Ausgang Q übertragen, wenn der Triggerimpuls 60 erscheint. Gleichzeitig wird der Durchschnittswert (dargestellt durch die unterbrochene Linie 62) der Abtastsignale A', B', C' und D', der am Eingang D des Speichers $L_5$ anliegt, an den Ausgang Q übertragen für den Vergleich mit den Abtastsignalen der nächsten nicht dargestellten Gruppe.

Obgleich die Erfindung beispielsweise mit der beschriebenen Schaltung realisiert werden kann, wird allgemein vorgezogen, einen Mikroprozessor oder Computer zu verwenden. Figur 5 stellt ein Flußdiagramm dar, das als Basis für Programme für Computer oder Prozessoren verwendet werden kann und insbesondere geeignet ist für das Modell HP9825A der Anmelderin. Die Zahlen innerhalb der Blöcke des Flußschaltbildes sind die Zahlen der entsprechenden Schritte im Programm.

Analogsignale werden durch eine Signalquelle 126 an einen A/D-Umsetzer 128 abgegeben, der zeitlich durch einen Taktgeber 130 gesteuert wird. Während einer ersten Gruppe von Abtastsignalen A, B, C und D wird in Programmschritten 2, 3 und 4 des Blocks 132 der Durchschnittswert der vorhergehenden vier Abtastsignale Z in Y eingegeben und ein neuer Durchschnittswert Z für die nächste Gruppe von Abtastwerten A', B', C' und D' berechnet. Auf diese Weise enthält die Speicherstelle Y jeweils den Durchschnittswert der vorherigen vier Abtastsignale. Das Abtastsignal einer zweiten Gruppe A', B', C' und D', welches ein möglicher Anzeigewert zu verschiedenen Punkten im Programm ist, wird in ein nicht dargestelltes Register DS eingesetzt. Wie schematisch durch Blöcke 134, 136 bzw. 138 angedeutet ist, wird dann bestimmt, ob $|A'-Y| > |B'-Y|$, ob $|A'-Y| > |C'-Y|$ und ob $|A'-Y| > |D'-Y|$. Das Programm schreitet von einem Block zum nächsten nur dann fort, falls die Antwort positiv ist. Falls alle Antworten positiv sind, wird der Abtastwert A' in das Anzeigeregister DS entsprechend Block 140 eingegeben und entsprechend Block 142 wird dieser Wert an die Anzeigevorrichtung 144 abgegeben. Falls die Antwort gemäß Block 134 negativ ist, so ist B' ein möglicher Anzeigewert und wird in das Register DS entsprechend Block 146 eingegeben, bis eine endgültige Entscheidung getroffen wurde. Aber bevor B' als Anzeigewert bestimmt ist, wird gemäß den Blöcken 148 und 150 bestimmt, ob $|B'-Y| > |C'-Y|$ und ob $|B'-Y| > |D'-Y|$ ist, wobei sich bestätigende Antworten ergeben müssen. Ist dieses der Fall, so wird der Abtastwert B', der sich bereits im Register DS befindet, in die Anzeigevorrichtung 144 gemäß Block 142 überführt. Falls jedoch die Entscheidung gemäß Block 148 negativ ausfällt, wird nunmehr der Abtastwert C' ein möglicher Anzeigewert, und er wird in das Register DS gemäß Block 152 eingegeben. Bevor dieser Wert jedoch als Anzeigewert ausgewiesen wird, muß entsprechend Block 154 bestätigt werden, daß die Relation $|C'-Y| > |D'-Y|$ gilt. Falls dieses zutrifft, wird der Abtastwert C', der sich bereits im Register DS befindet, an die Anzeigevorrichtung 144 gemäß Block 142 überführt. Falls die Entscheidung gemäß einem der Blöcke 150 oder 154 negativ ist, wird der Abtastwert D' in das DS-Register gemäß Block 156 eingegeben, und beim Abschluß des Verfahrens wird der Abtastwert D' in die Anzeigevorrichtung 144 gemäß Block 142 überführt.

Falls der Vergleich gemäß Block 134 positiv ausfällt, d.h. $|A'-Y| > |D'-Y|$ ist, jedoch die Entscheidung gemäß Block 136 negativ ist, d.h. $|A'-Y| < |C'-Y|$, kann der Abtastwert C' der Anzeigewert sein, und er wird somit in das Register DS gemäß Block 152 überführt. Das Programm fährt dann weiter vom Block 152 um zu bestimmen, ob entsprechend Block 154 in der vorher beschriebenen Weise $|C'-Y| > |D'-Y|$ ist. Ist dieses der Fall, so wird der Abtastwert C' an die Anzeigevorrichtung 144 vom Register DS überführt. Ist dieses nicht der Fall, so ist der Abtastwert D' der Anzeigewert und wird in das Register DS gemäß Block 156 eingegeben. Von dort wird der Abtastwert D' an die Anzeigevorrichtung 144 gemäß Block 142 überführt. Falls die Bedingungen gemäß den Blöcken 134 und 136 beide erfüllt sind und die Entscheidung gemäß Block 138 nicht erfüllt ist, d.h. $|A'-Y| < |D'-Y|$, ist der Abtastwert D' der Anzeigewert und wird als solcher gemäß Block 156 in das Register DS überführt. Von dort wird der Wert an die Anzeigevorrichtung 144 gemäß Block 142 überführt.

<u>Programm für HP Modell 9825A</u>

```
0:   "enhl":dsp "in enhl"
1:   for I=1 to 8192 by 8
2:   itf(K$[I,I+1])→rl; itf(K$[I+2,I+3])→r2
3:   itf(K$[I+4,I+5])→r3; itf(K$[I+6,I+7])→r4
4:   Z→Y; (rl+r2+r3+r4)/4→Z
5:   if abs(rl-Y)>abs(r2-Y); gto 10
6:   r2→C
7:   if abs(r2-Y)>abs(r3-Y); gto 13
8:   r3→C
9:   gto 18
10:  if abs(rl-Y)>abs/r3-Y); gto 16
11:  r3→C
12:  gto 18
13:  if abs(r2-Y)>abs/r4-Y); gto 21
14:  r4→C
15:  gto 21
16:  if abs(rl-Y)>abs(r4-Y); gto 20
17:  gto 14
18:  if abs(r3-Y)>abs(r4-Y); gto 21
19:  gto 14
20:  rl→C
21:  dsp C
22:  next I
```

Figur 6 zeigt eine EKG-Kurve, die unter Verwendung aller Abtastwerte gebildet wurde, die alle zwei ms als Anzeigewerte auftreten. Figur 7 zeigt wiederum eine EKG-Kurve, die aus den gleichen Eingangssignalen abgeleitet wurde, indem der Durchschnittswert jeder aufeinanderfolgenden und gegenseitig exklusiven Gruppe von vier Abtastsignalen als Anzeigewert gebildet wurde. Figur 8 zeigt die gleiche EKG-Kurve, die durch die Auswahl eines Abtastwertes in jeder folgenden und exklusiven Gruppe von vier Abtastwerten entsprechend der ersten Ausführungsform der Erfindung gebildet wurde. Der Schrittmacherimpuls P' in Figur 6 geht im Rauschen in der EKG-Kurve gemäß Figur 7 unter, jedoch wird dessen volle Amplitude in der EKG-Kurve gemäß Figur 8 beibehalten. Dieses ist natürlich von enormer Bedeutung bei der Überwachung der Funktion eines Herz- schrittmachers in einem Patienten.

Es versteht sich, daß die Datenreduktion nicht im Verhältnis 4:1 erfolgen muß. Wenn die Anzahl der Eingangssignale in einer Gruppe nicht eine Potenz von zwei ist, muß der Durchschnittswert der Abtastsignale in einer Gruppe durch eine Teilerschaltung bestimmt werden, und dieses kann nicht mehr einfach durch Vernachlässigung der bits mit niedrigerem Stellenwert erfolgen.

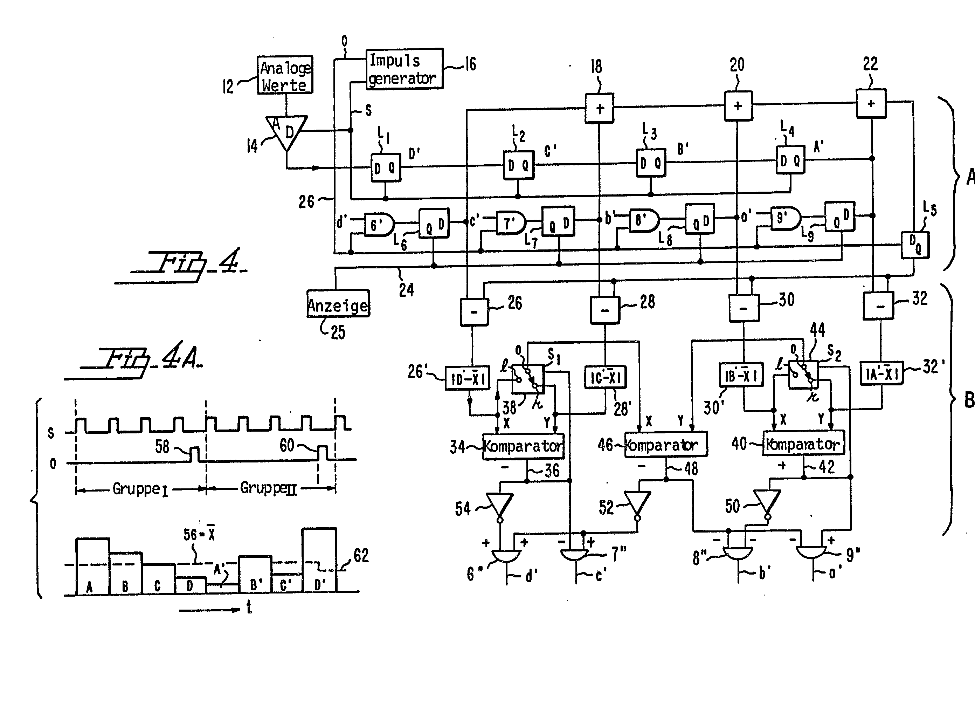

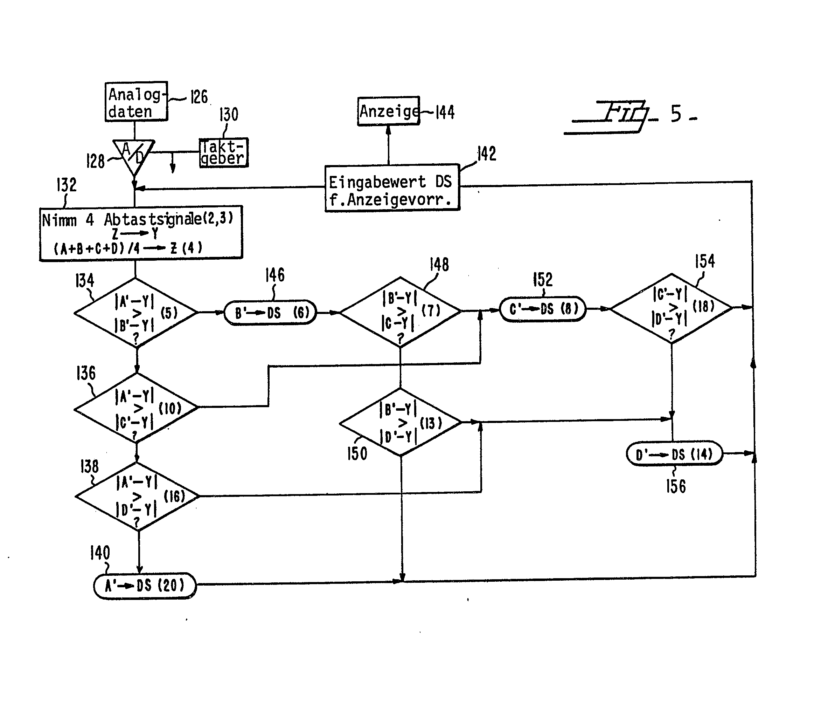

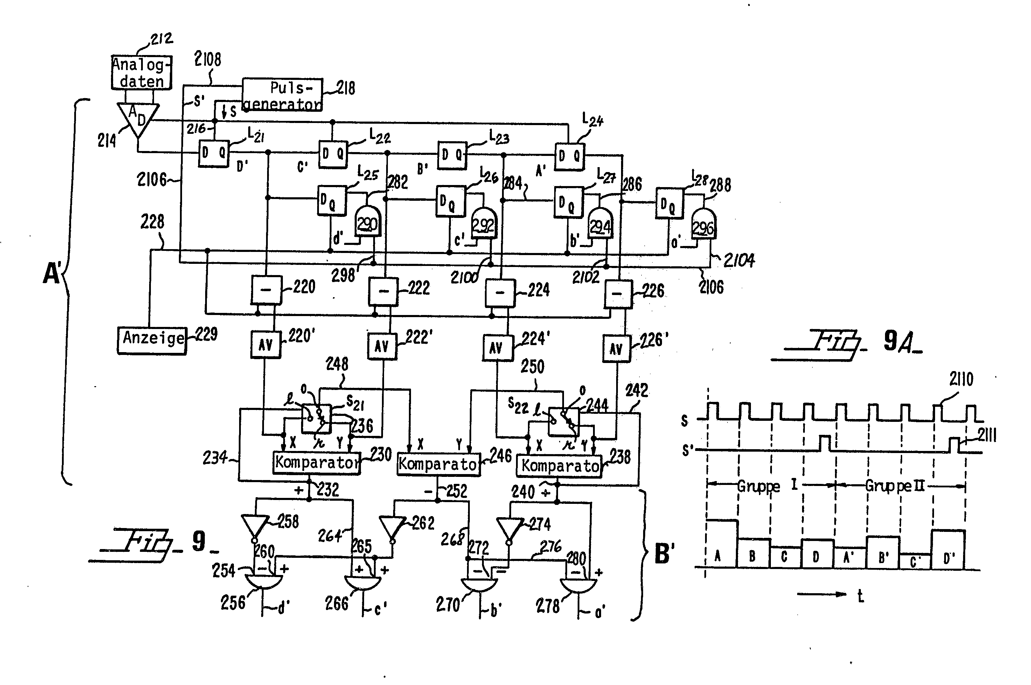

Es wird nun Bezug genommen auf die Schaltungsanordnung gemäß Figur 9 entsprechend einer anderen Ausführungsform der Erfindung. Hierbei wird als Anzeigesignal dasjenige Abtastsignal ausgewählt, welches sich von dem für die vorhergehende Gruppe ausgewählten Abtastsignal um den größten absoluten Betrag unterscheidet. Analogsignale, beispielsweise von einem EKG-Gerät, werden wiederum durch eine Signalquelle 212 einem A/D- Umsetzer 214 zugeführt, der digitale Abtastsignale einem Speicher $L_{21}$ zuführt, der in Reihe geschaltet ist mit Speichern $L_{22}$, $L_{23}$ und $L_{24}$. Der Ausgang Q jedes Speichers ist mit dem Eingang D des nächsten Speichers in Reihe geschaltet zur Bildung eines Schieberegisters. Der A/D- Umsetzer 214 und die Speicher $L_{21}$, $L_{22}$, $L_{23}$ und $L_{24}$ werden durch Takt- impulse S entsprechend Figur 9A betätigt, die durch eine Leitung 216 mittels eines Impulsgenerators 218 zugeführt werden. Die Ausgänge Q der Speicher $L_{21}$, $L_{22}$, $L_{23}$ und $L_{24}$ sind jeweils mit den Eingängen D der

Speicher $L_{25}$, $L_{26}$, $L_{27}$ und $L_{28}$ sowie mit den Eingängen der Subtrahierglieder 220, 222 , 224 und 226 verbunden. Die anderen Eingänge der Subtrahierglieder 220, 222, 224 und 226 sind mit einer Ausgangssammelleitung 228 verbunden zur Aufnahme des Abtastsignals der vorhergehenden Gruppe, das als Anzeigesignal ausgewählt wurde. Die Ausgangsleitung 228 ist mit einer Anzeigeeinrichtung 229 verbunden. Die Ausgänge der Subtrahierglieder 220, 222, 224 und 226 sind jeweils mit Schaltungen 220', 222', 224' und 226' zur Bildung des Absolutwertes verbunden. Die Ausgänge dieser Schaltungen 220' und 222' sind jeweils mit den Eingängen X und Y eines Komparators 230 sowie mit Eingangsklemmen 1 und r eines Schalters $S_{21}$ verbunden. Wenn der Wert am Eingang X kleiner als der Wert am Eingang Y ist, erzeugt der Komparator 230 einen Signalpegel H an dessen Ausgang 232. Der Ausgang 232 ist über eine Leitung 234 mit einem Anschluß 236 des Schalter $S_{21}$ verbunden. Wenn der Anschluß 236 den Signalpegel H hat, ist der Ausgang 0 des Schalters mit dessen Eingangsklemme r verbunden.

Die Ausgänge der Schaltungen 224' und 226' sind jeweils mit den Eingängen X und Y eines Komparators 238 sowie mit den Eingangsklemmen 1 und r eines Schalters $S_{22}$ verbunden. Wenn der Wert des Eingangs X kleiner als der Wert des Eingangs Y ist, erzeugt der Komparator 238 einen Signalpegel H an einem Ausgang 240. Der Ausgang 240 ist über eine Leitung 242 mit einem Anschluß 244 des Schalters $S_{22}$ verbunden. Wenn der Anschluß 244 den Signalpegel H hat, ist der Ausgang 0 des Schalters $S_{22}$ mit dessen Eingangsklemme r verbunden.

Die Ausgänge 0 der Schalter $S_{21}$ und $S_{22}$ sind jeweils mit den Eingängen X und Y eines Komparators 246 über Leitungen 248 und 250 verbunden. Wenn ein Wert an dessen Eingang X kleiner als der Wert an dessen Eingang Y ist, erzeugt der Komparator 246 einen Signalpegel H an dessen Ausgang 252.

Die Einrichtung zur Auswahl des Abtastsignales mit der größten absoluten Differenz von dem Abtastsignal der vorhergehenden Gruppe von Signalen kann die folgenden Logikschaltungen aufweisen. Ein Eingang 254 eines UND-Gliedes 256 ist über einen Umkehrverstärker 258 mit dem Ausgang 232 des Komparators 230 verbunden, und dessen anderer Eingang 260 ist über einen Umkehrverstärker 262 mit dem Ausgang 252 des Komparators 246 verbunden. Ein Eingang 264 eines UND-Gliedes 266 ist mit dem Ausgang 232 des Komparators 230 verbunden, und dessen anderer Eingang 265 ist über den Umkehrverstärker 262 mit dem Ausgang 252 des Komparators 246 verbunden. Ein Eingang 268 eines UND-Gliedes 270 ist mit dem Ausgang 252 des Komparators 246 verbunden, und dessen anderer Eingang 272 ist über einen Umkehrverstärker 274 mit dem Ausgang 240 des Komaparators 238 verbunden. Ein Eingang 276 eines UND-Gliedes 278 ist mit dem Ausgang 252 des Komparators 246 verbunden, und dessen anderer Eingang 280 ist mit dem Ausgang 240 des Komparators 238 verbunden.

Die Überleitung des ausgewählten Abtastsignales einer Gruppe an die Ausgangssammelleitung 228 und damit an die Anzeigeeinrichtung 229 geschieht folgendermaßen: die entsprechenden Ausgänge 282, 284, 286 und 288 von UND-Gliedern 290, 292, 294 bzw. 296 werden mit den Triggereingängen von Speichern $L_{25}$, $L_{26}$, $L_{27}$ und $L_{28}$ verbunden. Die Eingänge 298, 2100, 2102 und 2104 der UND-Glieder 290, 292, 294 bzw. 296 sind über eine Sammelleitung 2106 mit einem Ausgang 2108 des Impulsgenerators 218 verbunden, an welchem Ausgang der Ausgangstaktimpuls S' gemäß Figur 8A erscheint, und in nicht dargestellter Weise sind die entsprechenden Eingänge d', c', b' und a' der UND-Glieder 290, 292, 294 bzw. 296 verbunden mit den Ausgängen d', c', b' und a' der UND-Glieder 256, 266, 270 und 278. In noch zu beschreibender Weise führt nur einer der Ausgänge d', c', b' und a' den Signalpegel H, wenn ein Ausgangstaktimpuls bzw. Abtastimpuls S' auf der Sammelleitung 2106 erscheint, so daß nur eines der UND-Glieder 290, 292, 294 und 296 zwei Eingänge mit dem Signalpegel H hat und diesen Signalpegel am Ausgang hervorruft. Der Speicher $L_{25}$, $L_{24}$, $L_{27}$ oder $L_{28}$, mit dem dieser Ausgang verbunden ist, wird getriggert, um den Abtastwert vom Eingang D an dessen Ausgang Q und damit auf die Sammelleitung 228 und die Anzeigevorrichtung 229 zu übertragen. Der Abtastwert bleibt an diesen Punkten bis der Abtastpuls S' der nächsten Gruppe von Abtastsignalen auftritt, zu welchem Zeitpunkt der Sammelleitung 228 ein neues Abtastsignal zugeführt wird.

Die Funktion der Schaltung gemäß Figur 9 wird unter Bezugnahme auf die Signale S und S' der Figur 9A sowie anhand der Abtastwerte A, B, C und D der Gruppe I und A', B', C' und D' der Gruppe II beschrieben. Es wird angenommen, daß der Abtastwert A der Gruppe I als Anzeigesignal für diese Gruppe gewählt wurde. Nachdem der vierte Impuls 2110 des Abtastsignales S abgegeben wurde, befinden sich die Werte der Abtstsignale A', B', C' und D' an den Ausgängen Q der Speicher $L_{24}$, $L_{23}$, $L_{22}$ bzw. $L_{21}$. Diese scheinbare Umkehrung ergibt sich aus der Tatsache, daß der Abtastwert A' der erste in der Gruppe II ist, der aufzunehmen ist, so daß er alle Speicher $L_{21}$, $L_{22}$, $L_{23}$ und $L_{24}$ nacheinander durchläuft. Die Ausgangssignale der Subtrahierglieder 220, 222, 224 und 226 entsprechen D'-A, C'-A, B'-A bzw. A'-A und die Ausgänge der Schaltkreise 220', 222', 224' und 226' sind |D'-A| , |C'-A| , |B'-A| und |A'-A|. Es ist ersichtlich, daß |C'-A|>|D'-A| gilt, so daß am Ausgang 232 des Komparators 230 ein Signalpegel H (+) auftritt und bewirkt, daß der Ausgang O des Schalters $S_{21}$ mit dessen Eingangsklemme r verbunden wird, wie schematisch durch den Pfeil dargestellt ist, so daß der Wert |C'-A| an den Eingang X des Komparators 246 übertragen wird. Es ist weiterhin ersichtlich, daß |A'-A|> |B'-A| gilt, so daß der Ausgang 240 des Komparators 238 den Signalpegel H (+) aufweist und bewirkt, daß der Ausgang O des Schalters $S_{22}$ mit dessen Eingangsklemme verbunden wird, wie schematisch durch den Pfeil angedeutet ist, so daß der Wert |A'-A| dem Eingang Y des Komparators 246 zugeführt wird. Wenn die Bedingung |C'-A|> |A'-A| erfüllt ist, hat der Ausgang 252 des Komparators 246 einen Signalpegel L (-). Eine Überprüfung der sich ergebenden Zustände der Eingänge der UND-Glieder 256, 266, 270 und 278 zeigt, daß das UND-Glied 266 das einzige Logikglied ist, bei dem beide Eingänge den Logikpegel H aufweisen, so daß dessen Ausgang c' der einzige Ausgang mit dem Signalpegel H ist. Wenn daher der Ausgangsabtastimpuls 2111 der Gruppe II auftritt, hat der Ausgang des UND-Gliedes 252 den Signalpegel H und triggert den Speicher $L_{26}$. Der Speicher $L_{26}$ überträgt dann den Wert des Abtastsignales C' an dessen Ausgang Q und an die Ausgangssammelleitung 228, wo dieses Signal verbleibt, bis der nächste Impuls des Signales S' auftritt.

Das Ergebnis der Datenreduzierung gemäß diesem Ausführungsbeispiel ist, daß schmale impulsartige Signale mit relativ hoher Amplitude in der angezeigten Kurvenform nach der Datenreduzierung mit voller Amplitude erhalten bleiben.

Patentansprüche


1. Schaltungsanordnung zum Erzeugen einer kleineren Anzahl von Anzeigesignalen aus Eingangssignalen, welche relativ schmale Spitzenwertsignale, beispielsweise Herzschrittmacherimpulse, umfassen,
g e k e n n z e i c h n e t  durch eine erste Schaltung (A, A'),
welche die Eingangssignale in gleich große, einander ausschließende
Gruppen von jeweils mehreren Eingangssignalen zusammenfaßt, deren
Anzahl einem gewünschten Verhältnis von Eingangssignalen zu Anzeigesignalen entspricht, und jeweils in einer vorbestimmten, für jede
nachfolgende Gruppe gleichen Weise ein Referenzsignal ableitet von
den Eingangssignalen der vorherigen Gruppe und
eine zweite Schaltung (B, B') zur Aufnahme des jeweiligen Referenzsignales, welche zur Erhaltung der Spitzenwertsignale in der Anzeige
dasjenige Signal für Anzeigezwecke ableitet, dessen Amplitudenwert
die größte absolute Differenz bezüglich des für die vorhergehende
Gruppe abgeleiteten Referenzsignales aufweist.


2. Schaltungsanordnung nach Anspruch 1, dadurch  g e k e n n z e i c h -
n e t , daß das Referenzsignal von der ersten Schaltung (A) für eine
Gruppe von Eingangssignalen den Durchschnittswert der Amplituden aller
Signale dieser Gruppe von Eingangssignalen darstellt.


3. Schaltungsanordnung nach Anspruch 1, dadurch  g e k e n n z e i c h -
n e t , daß das Referenzsignal von der ersten Schaltung (A') für eine
Gruppe von Eingangssignalen dem Eingangssignal der jeweils vorhergehenden Gruppe entspricht, dessen Amplitudenwert die größte absolute
Differenz bezüglich einer Referenzamplitude aufweist, die in der
gleichen Weise von der Gruppe von Eingangssignalen ausgewählt wird,
welche der jeweils vorhergehenden Gruppe von Eingangssignalen vorhergeht.

1/4

0044436

Fig. 1

Fig. 2

Fig. 3

Fig. 6

Fig. 7

Fig. 8

Fig. 4.

Fig. 4A.

_FIG._ 5 _

_Fig_ 9_

_Fig_ 9A_

0044436

0044436

**Europäisches Patentamt**

## EUROPÄISCHER RECHERCHENBERICHT

Nummer der Anmeldung

EP 81 10 4947.7

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| A | DE – B1 – 2 454 839 (SIEMENS AG) <br><br> * Spalte 2, Zeile 13 bis Spalte 3, Zeile 32 * <br><br> -- | 1 | G 01 R 13/22 <br> G 01 R 13/34 <br> G 01 R 29/02 <br> A 61 B 5/04 |
| A | DE – B1 – 2 626 100 (SIEMENS AG) <br><br> * Spalte 2, Zeile 45 bis Spalte 4, Zeile 65 * <br><br> -- | | |
| A | MEDICAL AND BIOLOGICAL ENGINEERING, Vol. 12, No. 5 , September 1974 London <br> D.L. THOMAS et al. "A Vectorcardiograph for Assessing Implanted Cardiac Pacemakers" <br> Seiten 593 bis 598 <br> * Seite 594; Fig. 2; Seite 595, Absatz "Description of pacemaker vectorcardiograph" bis Seite 596 * <br><br> ---- | | |

**EINSCHLÄGIGE DOKUMENTE**

**RECHERCHIERTE SACHGEBIETE (Int. Cl.³)**

A 61 B 5/00
A 61 B 5/02
A 61 B 5/04
G 01 R 13/22
G 01 R 13/34
G 01 R 29/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 11-09-1981 | LEMMERICH |

EPA form 1503.1 06.78